# EUROPEAN PATENT APPLICATION

(11) **EP 3 940 705 A1**
(43) Date of publication of application: **19.01.2022**
(21) Application number: 20185396.7
(22) Date of filing: 13.07.2020
(51) Int. Cl.: G16B 40/20

(54) **METHOD FOR PREDICTION OF THE SILENCING EFFICIENCY OF GUIDES TARGETING A GENE OF INTEREST**

(71) Applicant: Helmholtz-Zentrum für Infektionsforschung GmbH, 38124 Braunschweig (DE)
(72) Inventor: Barquist, Lars, 97078 Würzburg (DE); Yu, Yanying, 97078 Würzburg (DE); Beisel, Chase, 97078 Würzburg (DE)
(74) Representative: Gramm, Lins & Partner Patent- und Rechtsanwälte PartGmbB

(57) **Abstract**

The present invention relates to a method for prediction of the targeting efficiency of guides targeting a gene of interest, said guides locating a target in a gene sequence, by evaluating of data provided by screens providing values of targeting characteristics of guides, characterized by the steps of
a) Selecting a set of guides along with gene intrinsic features related to a gene of interest and guide features;
b) Inputting the selected features into an automated machine learning algorithm and
c) Calculating an estimate of targeting efficiency of said selected guides by use of the automated machine learning algorithm.

## Description

The invention relates to a method for prediction of the targeting efficiency of guides targeting a gene of interest, said guides locating a target in a gene sequence by evaluating of data provided by screens providing values of targeting characteristics of guides.

The invention further relates to a computer program and a data processing apparatus arranged for performing the steps of this method.

The invention relates to a broad range of CRISPR technologies that impact the sequence, expression, or function of a gene-of-interest. These technologies rely on a CRISPR-associated (Cas) nuclease directed by a guide RNA (gRNA). The nucleases were derived from CRISPR-Cas systems, highly diverse immune systems found in bacteria and archaea. Beyond the nuclease, the gRNA directs the Cas nuclease to bind its nucleic-acid target through extensive base pairing between the target and the guide portion of the gRNA. In most cases, the target must be flanked by a protospacer-adjacent motif (PAM), a protospacer-flanking sequence (PFS), or sequence lacking strong complementarity to the "handle" of the repeat sequence within the gRNA. Upon recognition, the nuclease exhibits different activities specific to the nuclease, such as introducing a blunt double-stranded break in DNA (e.g. Cas9), introducing a staggered double-stranded DNA break (e.g. Cas12), degrading the target strand of double-stranded DNA in the 3' to 5' direction (e.g. Cascade-Cas3), cleaving single-stranded DNA (e.g. Cas9, Cas12), cleaving RNA and DNA from a transcriptional active locus (e.g. Csm, Cmr), or cleaving RNA (e.g. Cas9, Cas13). Target recognition can also elicit non-specific cleavage of single-stranded DNA or RNA depending on the nuclease.

These activities can also be eliminated by mutating the responsible endonuclease domain (e.g. HNH, RuvC, HEPN) or, in the case of nucleases comprised of multiple protein subunits, removing the subunit with endonucleotlytic activity (e.g. Cas3). These changes allow the nuclease to recognize and bind the target without cleaving or degrading the target. As a result, the nuclease can be used to interfere with the function of the target (e.g. blocking transcription) or recruit other factors to modify the target (e.g. epigenetic modulators, chemical-modifying domains). These capabilities have opened a wide range of applications for CRISPR technologies that includes genome editing, gene regulation, gene drives, RNA modifications, antimicrobials, in vitro diagnostics, non-invasive imaging, programmable restriction enzymes, genetic recording devices, and more.

CRISPR technologies were first introduced for editing DNA sequences through DNA cleavage and subsequent repair. Repair could occur through non-homologous end joining or homology-directed repair. More recent technologies have involved base editors that chemically modify DNA bases (e.g. through the action of cytidine deaminases) within the target sequence as well as prime editors that provide template-mediate editing of a small sequence within and adjacent to the target sequence. These technologies have been implemented in phages, viruses, bacteria, and lower and higher eukaryotes. The efficiency of gene editing can vary widely for each mode of editing and can depend on various factors, such as the GC-content of the target sequence or the presence of internal PAMs.

CRISPR interference (CRISPRi) is a separate technology in which a catalytically-dead Cas protein (for example dCas9, dCas12a) is directed to a gene of interest, where it binds, blocking transcription and leading to gene silencing. CRISPRi has become a prevalent technique in bacteria for studying the function of individual genes, regulating pathways for metabolic engineering and performing genome-wide genetic screens. In eukaryotes, binding DNA is typically not sufficient to block transcription. However, adding domains that block RNA polymerase or modulate the epigenetic state can enhance silencing, such as by recruiting the KRAB domain. Whether in bacteria or eukaryotes, silencing by CRISPRi is generally variable and not complete, and silencing efficiency is potentially affected by a range of factors, including basal gene expression, guide: DNA hybridization, and sequence features of the guide and targeted region.

CRISPR activation (CRISPRa) is a complementary technology to CRISPRi that results in gene activation rather than interference. Here, the catalytically-dead nuclease recruits a domain that in turn recruits RNA polymerase or drives an epigenetic state more conducive to transcription. CRISPRa has been implemented in bacteria and eukaryotic cells, including yeast, mammalian cells, and plants.

A separate application of CRISPR technologies involves targeting RNA. The most common example is with the nuclease Cas13, which naturally targets RNA as part of its role in antiviral defense. This nuclease was co-opted for programmable RNA targeting in eukaryotes, where it's used as an analog for RNA interference. Cas13 has been extended for genome-wide screens. Separately, mutating the HEPN domain renders Cas13 catalytically dead and allows it to bind but not cleave DNA. This in turn has been employed for RNA imaging when attaching a fluorophore or for RNA editing when attaching an RNA modification domain.

Across these examples and the many other CRISPR technologies, there is a need for a design tool for selection of guides targeting a gene of interest. This allows to optimize guide RNAs. A number of design tools have been developed for guide selection that range from identifying sites flanked by a PAM to accounting for additional factors such as GC content or propensity for off-target effects. The most advanced tools have implemented machine learning to develop algorithms that predict guide efficiency based on a combination of factors. These approaches have relied on high-throughput datasets, such as canvasing a single gene for all possible target sites or targeting multiple genes across a genome.

In these cases, the relevant features have specifically focused on the target or guide sequence, such as its GC content, local folding, or presence of certain sequence motifs. However, these algorithms fail to account for gene-specific factors that could influence guide efficiency, such as their transcriptional activity or length.

M. Haeussler, J.-P. Concordet: Genome Editing with CRISPR-Cas9: Can It Get Any Better?, in: J Genet Genomics. 2016 May 20; 43(5): 239-250 describes a guide RNA design tool developed based on eukaryotic data in CRISPR-Cas9.

F. Rousset, L. Cui, E. Siouve, C. Becavin, F. Depardieu, D. Bikard: Genome-wide CRISPR-dCas9 screens in E. coli identify essential genes and phage host factors, in: PLOS Genetics, November 7, 2018, p. 1-28 discloses a result of data obtained from a CRISPR-dCas9 screen related to *E*. *coli* strains considering the target strand and the template strand of the gene of interest after bacterial growth. A library of guides and their fitness effects is obtained.

A. Calvo-Villamañán, J. Wong Ng, R. Planel, H. Menager, A. Chen, L. Cui, D. Bikard: On-target activity predictions enable improved CRISPR-dCas9 screens in bacteria, in: Nucleic Acids Research, 2020, p. 1-8 describes a model to predict the ability of guide RNAs to direct dCas9 to block the RNA polymerase (RNAp) elongation in E. *coli* when binding to the non-template strand (coding strand). An importance of bases surrounding the PAM sequence, bases in the seed sequence and to lower degree bases up to 16nt downstream of the PAM was found.

G. Liu, Y. Zhang, T. Zhang: Computational approaches for effective CRISPR guide RNA design and evaluation, in: Computational and Structural Biotechnology Journal 410, December 3, 2019 describes tools for CRISPR guide RNA design using hypothesis-driven methods and learning-based methods. These tools predict the specific key of guide RNAs aligned to a given to predict the cutting specificity and return of-target sequences and sites, i.e. a preferred position of cutting a gene strand and implementing the guide RNA. It was found that the major outcomes of Cas9 cleavage is non-random and predictable. However, tools for large indels, homozygous sites and the activity of nucleases other than SpCas9 are required.

H.-H. Wessels, A. Mendez-Mancilla, X. Guo, M. Legut, Z. Daniloski, N.E. Sanjana: Massively parallel Cas13 screens reveal principles for guide RNA design, in: Nature Biotechnology 2020, pages 722-727 describes a random forest model trained on data derived from targeting a single gene, and using guide sequence and thermodynamic features. The consequences of gene effects for targeting are not considered. P.C. Despres, A.K. Dube, M. Seki, N. Yachie, C.R. Landry: Perturbing proteomes at single residue resolution using base editing, in: Nature Communications 2020, article number 1871 describes targeting essential genes in the yeast *Saccharomyces cerevisiae* using a Cas9 base editor. Measurements of guide depletion are used to identify sequence and thermodynamic features that effect editing efficiency. A predictive model is not created, and gene effects on editing efficiency are not considered.

J.G. Doench, E. Hartenian, D.B. Graham, Z. Tothova, M. Hegde, I. Smith, M. Sullen-der, B.L. Ebert, R.J. Xavier, D.E. Root: Rational design of highly active sgRNAs for CRISPR-Cas9-mediated gene inactivation, in: Nature Biotechnology 2014, pages 1262-1267 describes a support vector machine algorithm for Cas9 gene inactivation activity in eukaryotes using training data from targeting 8 genes. Guide sequence features and GC content were considered.

An object of the present invention is to provide an improved method for prediction of the targeting efficiency of guides targeting a gene of interest and a related computer program and data processing apparatus.

The object is achieved by the method comprising the steps of claim 1 and the related computer program according to claim 12 and the data processing apparatus according to claim 13.

The method according to the present invention comprises the steps of
a) Selecting a set of guides along with gene intrinsic features related to a gene of interest and guide features,
b) Inputting the selected features into an automated machine learning algorithm and
c) Calculating an estimate of targeting efficiency of said selected guides by use of the automated machine learning algorithm.

It was found that the use of a set of features, which comprises two different types including gene intrinsic features related to the gene of interest, which are not modifiable and fixed by the characteristic of the gene of interest, and guide features, which are modifiable in the design process, allows an improved training and use of an automated machine learning algorithm in order to predict the targeting efficiency of guides targeting the gene of interest.

In particular when using CRISPRi as an exemplary case study, it was found that these factors can represent confounding variables that reduce the predictive power of the resulting algorithm when training on genome-wide screening data. However, through iterative training on guide-specific features and gene-specific features, gene-specific effects can be removed leading to a more accurate prediction of guide efficiency. This approach can be applied for guide design not only for CRISPRi in bacteria but also the many other CRISPR technologies in bacteria in eukaryotes. The only requirement is a large-scale screen performed across multiple genes. The resulting data then serves as input to the method for training the final algorithm. Users can then employ the algorithm to select the guide RNAs with the greatest predicted activity, whether designing individual guide RNAs, sets of guide RNAs for multiplexed targeting, or entire libraries for genetic screens.

The term "targeting a gene" is to be understood broadly in the sense, that the guides target a transcribed region of a gene, a promotor of a gene, a transcript of the gene, i.e. the RNA or a part of it, and the like. Targeting can also occur outside of the promoter, such as when altering the epigenetic state of the genetic locus. Targeting can result in down-regulation or up-regulation of the gene as well as changes to the DNA or RNA that alters the sequence and function of the resulting protein.

The automated machine learning algorithm is driven by selecting a set of guides from a larger set of guides provided by e.g. CRISPRi screening along with guide features related to the selected guides. It was found that the outcome of the automatic machine learning algorithm to predict the targeting efficiency of guides targeting a gene of interest highly depends on the selection of features inputted into the machine learning algorithm. In particular by using a set of features comprising a first set selected from gene intrinsic features and a second set selected from guide features, two different models are achieved, wherein the first model considers the effect of the guide features modifying the design process and the second model considers the effect of the gene features, which are not modifiable and relate to the gene of interest itself. The automated machine learning algorithm then combines both models with the result of an improved prediction of the depletion of guides targeting a gene of interest.

The method makes use of an evaluation of data provided by screens, which provide targeting charabcteristics of guides. Preferably, CRISPRi screens can be used for bacteria. However, other techniques for screens are also applicable as well as other types of organisms. For example, the method can also be based on the evaluation of data provided by gene disruption, in particular when targeting eucariotic genes. These could include gene disruption through formation of random insertions or deletions (indels) via DNA cleavage or through the introduction of defined disruptive mutations (e.g. premature stop codon, frameshift, altered splicing site, altered stability, lost protein function) through homology-directed repair, base editors, or prime editors. These disruptions would then be associated with lethality under all conditions (i.e. essential genes) or under specific conditions (i.e. conditionally essential or important). Genes could also be downregulated at the transcriptional level by blocking transcription or altering the epigenetic state of the genetic locus. Genes could also be disrupted at the post-transcriptional level by cleaving the mRNA with RNA-targeting nucleases such as Type III CRISPR-Cas systems, Cas13 nucleases, or certain Cas9 nucleases (e.g. CjeCas9, NmeCas9, SauCas9). RNA-modifying enzymes could also be employed to block translation of the full-length protein, destabilize the mRNA, or introduce deleterious mutations. Finally, as the screens only require a change in fitness, they could include also be coupled with gene activation such as through CRISPR activation (CRISPRa). These screens would involve upregulating expression that permits growth (e.g. conferring drug resistance) or reduces growth (e.g. metabolic imbalance from protein overexpression).

The method can be used for predicting guide efficiency for the purposes of both targeting single genes, as well as designing multi-gene or genome-wide libraries.

The method provides a list of predicted targeting efficiencies of guides for specific targets, which can be used for selecting e.g. the best fitted guides for a following process of targeting a gene of interest with the at least one selected guides. For example, individual guide RNAs can be selected that are predicted to silence a gene-of-interest in bacteria to study its contribution to cell physiology. The user would then not need to screen large numbers of guide RNAs to find ones with strong silencing efficiency. Separately, sets of efficient guide RNAs could be designed for silencing genes associated with competing metabolic pathways as part of strain engineering and biochemical production. The algorithm could even be applied to design genome-wide screens, where testing individual guides would be infeasible. Finally, aside from selecting guides predicted to exhibit the highest efficiency, the algorithm could be implemented to select guides that exhibit moderate or even low silencing activity in order to partially downregulate a gene-of-interest. This capability could be important e.g. when probing the physiological contributions of essential genes. Similarly, with corresponding high-throughput datasets, an algorithm could be developed to predict formation of disruptive indels, base edits, prime edits, or effective antimicrobials.

Preferably, in addition to the known features used in prior art methods for prediction of the depletion of guides targeting a gene of interest, a plurality of additional guide features is selected comprising thermodynamic, genomic, and transcriptomic features. Said set of features can comprise sequence features comprising guide RNA and PAM single nucleotide sequences and 30nt extended sequence dinucleotide features, thermodynamic features including minimum free energy of guide RNA monomer, guide RNA homodimer, and targeting DNA and guide RNA hybrid, as well as genomic features describing gene and operon organization, e.g. distance to start codon. In addition, transcriptomic features can be used describing the gene expression level. Including these features helps to assess the structural stability upon target recognition, the accessibility of guide RNA, polar effects and other confounding effects.

The automated machine learning algorithm comprises parameters, which can be trained by calculating at least one error value by comparing the calculated level of depletion of a set of selected features with a known level of depletion of said selected features, adapting the parameters as a function of at least one calculated error value, and iteratively repeating the step of calculating a level of depletion with the automated machine learning algorithm in order to adapt the parameters for reducing the at least one error value.

A predictive model for guide efficiency is provided by first training and later use of the trained automated machine learning algorithm. The use of the automated machine learning algorithm may optionally come with a continued training of the predictive model.

Preferably, in the first step a predictive model is trained to predict guide depletion in essential genes in at least one genome wide screen using feature which can not be modified in guide design (so-called gene features, e.g. basal expression, operon structure). In a second step, a second random forest regression model is trained on the residual depletion using features which can be modified in guide design (so-called guide features, i.e. the location of the guide and therefore the sequence composition and thermodynamic properites). The order of the first and second step is arbitrary. This procedure is iterated until the joint prediction of the two models is optimized. This work flow can be performed by use of a computer program and can be applied to any bacterium, eukaryote, or the like where a genome-wide screen providing targeting characteristics for guides is available.

For example, a genome-wide screen could have been performed on essential genes by inputing the depletion score for each guide sequence. The screen could also be focused on genes critical only for a specific growth condition, such as growth on minimal medium but not nutrient-rich medium, resulting in a similar set of depletion scores. A similar screen could also be conducted with Cas13, when targeting relies on induction of collateral activity that drives dormancy in bacteria. This could involve targeting non-essential genes, where gene-specific factors such as expression level may impact the onset of dormancy of subsequent guide depletion in the population.

Separately, genome-wide screens could be conducted that enrich efficient guide sequences. For example, genes that confer drug resistance when disrupted could serve as one enrichment-based screen. The screen could also be conducted with CRISPRa by targeting genes that confer stress tolerance when overexpressed. Here, each gene would be expected to confer different extents of tolerance that would form the basis of a gene-specific factor.

The output of the model for prediction of guide efficiency is a ranked list of optimal guides for targeting that gene. Preferably, the model drives a software tool to select optimal guides for genome-wide libraries. The software tool can be arranged to provide the guide sequences, a score predicting targeting efficiency, and an explanation of the factors effecting the efficiency score.

Preferably, the step of adapting the parameters is carried out by Random Forest Regression. This provides improved results compared to Linear Regression.

The quality of the prediction of the guide efficiency can be improved by separate handling of the gene-intrinsic effects from guide effects that can be manipulated in design. Therefore, it is preferred when the automated machine learning algorithm preferably comprises two models separately trained from each other, wherein the first of the two models using the selected gene intrinsic features and the second of the two models using the selected guide features. Thus, the first model can be adapted to use the gene intrinsic features and the second model can be adapted to use the guide features, wherein the first model is trained with the gene intrinsic features, but not with the guide features, and the second model is trained with the guide features, but not with the gene intrinsic features.

Preferably, the steps of training the parameters of the automated machine learning algorithm is done with at least two sets of CRISPRi screening data, which are obtained by different methods.

This can be implemented by use of a first set of screening data, which first set is obtained by only including guide RNAs for targeting the coding strand of the gene of interest and collected in only one specific growth phase, and a second set of CRISPRi screening data which second set is obtained by including guide RNAs for targeting the coding strand and the template strand of the gene of interest.

The method may comprise the additional step of classifying the guide RNA provided in at least one set of screening data, which is used for training of the parameters of the automated machine learning algorithm, into a group of guide RNAs providing strong depletion and a group of guide RNA providing weak depletion. Classifying the guide RNA can be used for the selection step and results in a reduced prediction error.

The set of guide features selected in step a) may comprise the feature of an RNA expression level, and/or the feature of protein expression.

The gene intrinsic features selected in step a) for including in the set of features preferably comprises the locus tag, the length of the targeting gene of interest, the guanine-cytosine (GC) content of the targeting gene of interest, the distance of the targeting gene of interest to start of an operon, the distance of the targeting gene of interest to start of the operon relative to the operon length, the number of downstream genes in the same operon, the number of downstream essential genes in the same operon, the minimal expression level from a first concentration to a second concentration of cells when the cells were collected, and the maximal expression level from a first concentration to a second concentration of cells when the cells were collected.

The guide features selected in step a) for including in the set of features preferably comprises the guanine-cytosine (GC) content of the guide RNA, the distance to start a codon, the distance to start the codon relative to the length of the gene of interest, the function of a guide RNA of being able to target a coding strand, the function of a guide RNA being able to target intergenic regions, the length of the longest consecutive identical nucleotides, the minimum free energy of hybridization of targeting DNA and guide RNA, the minimum free energy of hybridization of a specific region of targeting DNA and guide RNA, the minimum free energy of guide RNA homodimer, the minimum free energy of guide RNA monomer, the number of potential of-targets with specific similarities, at least one PAM sequence, at least one guide RNA sequence, and a plurality of possibilities for extended sequence dinucleotides.

The method can be implemented in a computer program comprising instructions which, when the program is executed by a computer, cause the computer to carry out the steps of the above described method

The object is further achieved by a data processing apparatus comprising means for carrying out the steps of the above described method.

In the following, the invention is explained by way of an exemplary embodiment with the enclosed drawings. It shows:
- Figure 1 -: Flow diagram of the method for prediction of the targeting efficiency of guides targeting a gene of interest, said guides locating a target in a gene sequence;
- Figure 2 -: Flow diagram of the training session iteratively performed for a gene model and a guide model in a successive chain;
- Figure 3 -: Diagram of the qualitative differences in the log2FC distribution of guide RNAs for essential genes for two different sets of CRISPRi screens;
- Figure 4 -: Explanation of the genomic and sequence features;
- Figure 5a) -: Diagram showing the effect of the ensemble size on the Spearman correlation;
- Figure 5b) -: Diagram showing the effect of the ensemble size on the mean absolute error of prediction;
- Figure 6 -: Diagram of the Spearman correlation of the autocorrelation of gene features and guide intrinsic features over the number of iterations;
- Figure 7 -: Diagram of the Spearman correlation of the autocorrelation of gene features and guide intrinsic features, the sum of the two models, the validation of the summed model and the validation of the summed model with the first set of CRISPRi screen and with the the second set of CRISPRi screen over the number of iterations;
- Figure 8 -: Diagram of the experimental log2FC value compared to the known log2FC for guide features performing well versus guide features performing poorly based on clustering the logFCs;
- Figure 9 -: Diagram of the true positive rate over the false positive rate using different sets of CRISPRi screen data;
- Figure 10 -: Table of the SHAP values of summed model with the feature "coding strand";
- Figure 11 -: Table of the Gini importance of the 10 most relevant gene intrinsic features and guide features for E. coli related embodiment;
- Figure 12 -: Table of the mean decrease accuracy of the 10 most relevant gene intrinsic features and guide features for E. coli related embodiment;
- Figure 13 -: Table of the Gini importance of the 10 most relevant gene intrinsic features for E. coli related embodiment excluding the coding strand features after 17 iterations;
- Figure 14 -: Table of the mean decrease accuracy of the 10 most relevant gene intrinsic features for E.coli related embodiment excluding the coding strand features after 17 iterations;
- Figure 15 -: Table of the Gini importance of the 30 most relevant guide features for E. coli related embodiment excluding the coding strand features after 17 iterations;
- Figure 16 -: Table of the mean decrease accuracy of the 30 most relevant guide features for E. coli related embodiment excluding the coding strand features after 17 iterations;
- Figure 17 -: Table of the Gini importance of the 10 most relevant gene intrinsic features for E. coli related embodiment excluding the coding strand features after 17 iterations;
- Figure 18 -: Table of the mean decrease accuracy of the 10 most relevant gene intrinsic features for E. coli related embodiment excluding the coding strand features after 17 iterations;
- Figure 19 -: Table of the Gini importance of the 30 most relevant guide features for E.coli related embodiment excluding the coding strand features after 17 iterations;
- Figure 20 -: Table of the mean decrease accuracy of the 30 most relevant guide features for E.coli related embodiment excluding the coding strand features after 17 iterations.
- Figure 21 -: Plot showing a comparison of log fold-changes in luminescence experimentally determined determined by flow cytometry in *Escherichia coli* to predictions of guide efficiency from our algorithm (CRISPRi) and a selection of other potential methods for predicting guide efficiency.
- Figure 22 -: Plot showing a comparison of log fold-changes in luminescence experimentally determined determined by flow cytometry in *Salmonella enterica* to predictions of guide efficiency from our algorithm (CRIS-PRi) and a selection of other potential methods for predicting guide efficiency.

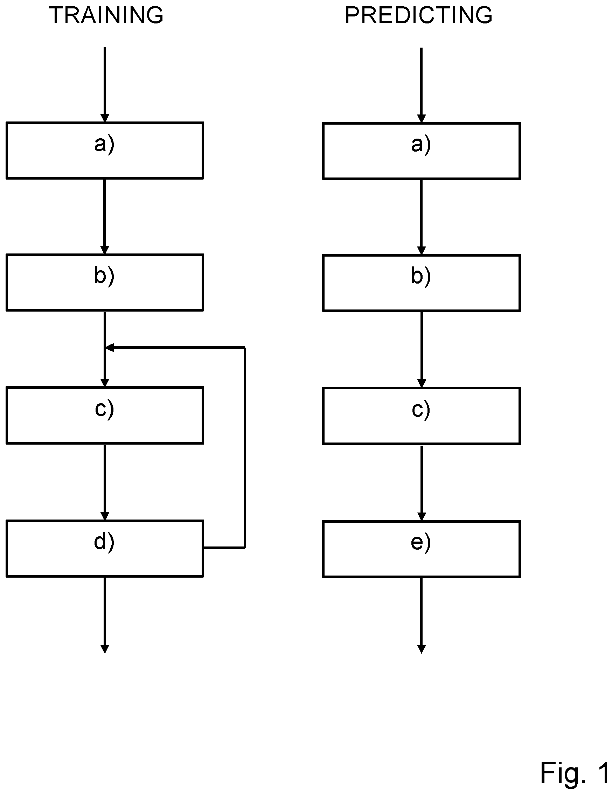

Figure 1 shows a flow diagram of the method for prediction of the targeting efficiency of guides targeting a gene of interest, said guides locating a target in a gene sequence by evaluating of data provided by Clustered Regularly Interspaced Short Palindromic Repeats Interference (CRISPRi) screens as an example.

The method comprises the steps of
a) Selecting a set of guides along with gene intrinsic features related to a gene of interest and guide features,
b) Inputting the selected features into an automated machine learning algorithm and
c) Calculating an estimate of targeting efficiency of said selected guides by use of the automated machine learning algorithm.

These steps are performed for the training session for the parameters, i.e. the model of the automated machine learning algorithm making use of known prediction results. The same steps are performed for the prediction session making use of an already trained automated machine leraning algorithm in order to predict the depletion of guides, i.e. the siliencing efficiency of guides, with unknown result.

In the training session, in a step d), a training of parameters of the automated machine learning algorithm is performed by calculating at least one error value by comparing the calculated estimate of targeting efficiency (i.e. the level of depletion) of a set of selected guides with a known targting efficiency of said selected guides and adapting the parameters as a function of the calculated error value. These sequence of steps c) and d) are iteratively repeated in order to adapt the parameters for reducing the at least one error value. The number of iterations can be set according to experiences with the convergence behavior of the trained model.

In the prediction session, the result is given out, e.g. on a display, as print out or data set, in step e). This output of the model for prediction of guide efficiency can be a ranked list of optimal guides for targeting that gene. The output can provide the guide sequences, a score predicting targeting efficiency, and an explanation of the factors effecting the efficiency score. The output can also drive a software tool to select optimal guides for genome-wide libraries.

The method can be applied, for example, to accurately predict guide depletion, i.e. the targeting efficiency, in (publicly available) CRISPRi essentiality screens in *Escherichia coli,* using a variety of sequence, thermodynamic, and transcriptomic features applied to an automated machine learning algorithm. In an iterative procedure targeting activity can be separated from confounding effects, for instance operon structure and gene expression. The resulting design algorithm shows an improved prediction accuracy compared to existing design tools developed for genome editing in predicting GFP targeting efficiencies. The method provides insight into guide features affecting CRISPRi silencing. The method can also be applied to other screens, such as essentiality screens based on formation of deleterious indels. The method can also be applied to screens associated with guide enrichment, such as when screening for gene disruptions that promote drug resistance, or when screening for overexpression phenotypes associated with stress tolerance.

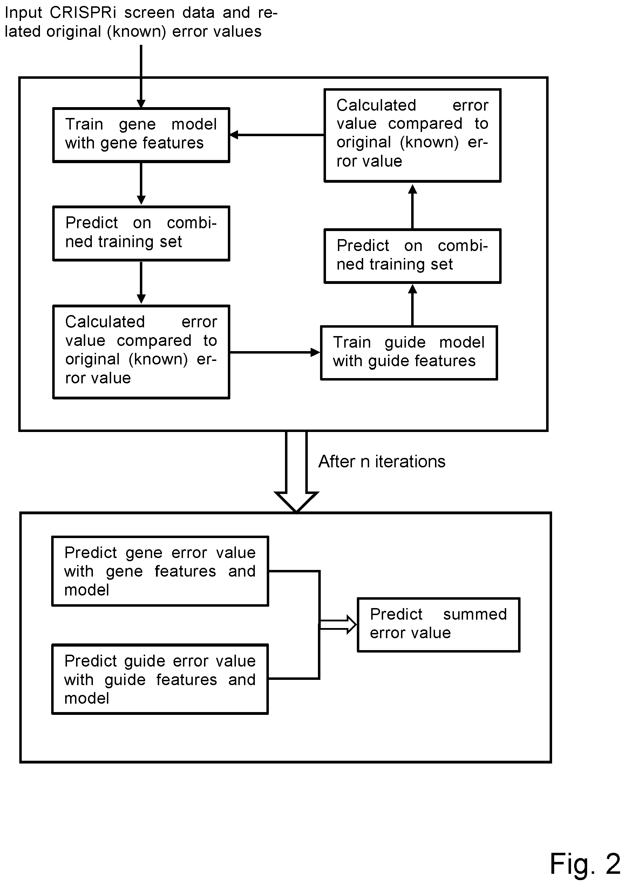

Figure 2 shows a flow diagram of the training session iteratively performed for a gene model and a guide model in a successive chain. The training is performed by inputting CRISPRi screen data and related original (known) depletion values into an automated machine learning algorithm comprising a gene model related to gene intrinsic features and a guide model related to guide features. The gene model is trained with gene features and a combined training set is predicted for this model. The error value is calculated by comparing the error value of the predicted combined training set with the known original depletion value. Then, the guide model is trained with guide features and a combined training set is predicted for this model. The error value for the guide model is calculated by comparing the error value of the predicted combined trainig set with the known original error value from the gene model. This process is iteratively repeated for a given number of times.

After a number of n iterations, the resulting gene error value is predicted with the gene features and the gene model. Accordingly, the resulting guide error value is predicted with the guide features and the guide model. Both, the gene error value and the guide error value are summed up in order to predict a summed error value.

The above mentioned error values can be e.g. the residual log2FC.

### Training Dataset

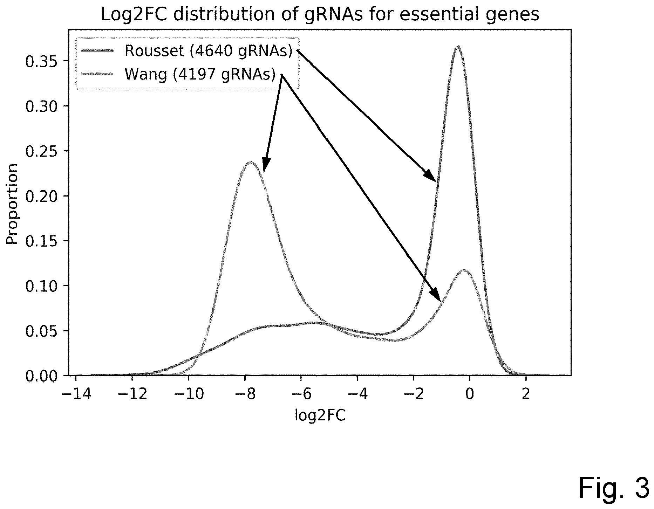

Figure 3 shows a diagram of the qualitative differences in the log2FC distribution of guide RNAs for essential genes for two different sets of CRISPRi screens.

In an exemplary embodiment, the data from two previous CRISPRi genome-wide essentiality screen studies in *E.coli* K12 MG1655 were collected.

Given that genome-wide screening data present less batch effects, guide RNA data for essential genes had been used from two CRISPRi genome-wide essentiality screens in *E.coli* K12 (Wang et al., Pooled CRISPR interference screening enables genome-scale functional genomics study in bacteria with superior performance, in: Nature Communications 9, Art,-No. 2475 (2018) and Rousset et al., Genome-wide CRISPR-dCas9 screens in E. coli identify essential genes and phage host factors, in: PLOS Genetics, November 7, 2018, p. 1-28). The methods of two CRISPRi screens differed. The first method used by Wang only included guide RNAs for targeting coding strand while the second method used by Rousset included guide RNAs for targeting both the coding stand and the template stand. Cells were collected at different growth phases in each study. The resulting log2 fold change (log2FC) distribution exhibited qualitative differences as shown in Figure 3.

The sequence, targeting gene, gene position and fitness effect of each guide RNA are available as supplementary documents. The sequence and gene position were curated to the latest version reference genome (NC_000913.3). Guide RNAs identified as bad quality or with sequences that differ from the reference genome due to different versions had been discarded. 8837 guide RNAs for essential genes were extracted from each dataset and merged to form one dataframe, among which 49% (0.7*0.7) were used for training, 21% (0.7*0.3) for testing, and 30% for validation. This data fusion increased the sample size and improved the model robustness by incorporating differences among datasets.

Machine learning was then used as a powerful method to predict guide RNA efficiency by learning from these two massive sets of CRISPR screen data and numerous features. As the learning target, the readout of CRISPR screen-depletion-presumably combines actual gene fitness effect and guide RNA efficiency, thus studying essential genes untangles guide RNA efficiency given the theoretically infinite fitness effect. The key of learning lies in the features.

The model performance considerably depends on feature preprocessing, model selection, and parameter tuning. While manual optimization can be error-prone, applying automated machine learning simplifies the process and provides better outcomes. And the lack of model explanation in previous studies failed to elucidate the importance of features thus limiting the improvement and simplification of the model and future exploration of mechanisms.

A robust machine learning-based design tool is achieved by the above shown sequence with an extended feature set using automated machine learning and extract explicit design rules based on feature contributions from the predictive model.

### Feature Engineering

Feature engineering can be performed by use of a software script to compute a number of e.g. 580 sequence, thermodynamic, genomic, and transcriptomic features. Sequence features including 556 single nucleotide and dinucleotide features were one-hot encoded. Thermodynamic features including minimum free energy for different structures can be computed using e.g. the known software tools ViennaRNA Package 2.0 and RNAduplex for RNA:RNA hybrid, RNAduplex for DNA:RNA hybrid and RNA-fold for RNA folding. Genomic features including gene and operon organizations can be based on the reference genome, essential gene list in e.g. LB Lennox medium from EcoCyc, and operon dataset from RegulonDB. Transcriptomic feature data including gene expression levels in a number of e.g. 10 different ODs can be obtained from previous studies. Minimal or maximal expression levels can be extracted in a given range of ODs according to the growth phase when cells were collected: OD 0.1 - 1.4 for Wang dataset; and all ODs for Rousset dataset. Off-targets can be counted by alignment to all possible guides with NGG PAM in reference genome using blastn (with parameter -task "blastn-short") in BLAST+ (version 2.6.0)

### Applying automated machine learning

The method can be applied on the automated machine learning algorithm provided e.g. with the software toolkit *auto-sklearn,* which can be implemented in a software script to develop an optimized machine learning regression model. All possible estimators and preprocessors should be included. Followed parameters can be used to set up the toolkit *auto-sklearn:* time_left_for_this_task=3600, per_run_time_limit=360, resampling_strategy= 'cv', and resampling_strategy_arguments={'fold':5}. If not specified, parameters can be the same as default. A given number of e.g. 10 ensemble sizes should be tested and the model including imputer, variance threshold selector, and scaler optimized with ensemble size 1 is further evaluated and interpreted using e.g. the software tool *scikit-learn* and SHAP (SHapley Additive exPlanations). Receiver operating characteristic (ROC) curves can be computed using different percentiles of log2FC values as thresholds. Area under the ROC curve (AUC) should be used to evaluate the classification accuracy.

### Segregation of guide and gene effects

Guide-specific and gene-specific features are divided and, as shown in Figure 3, gene-specific features should be firstly used to train a gene model. In each iteration, residual log2FC should be calculated by subtracting the predicted log2FC by gene or guide model from the experimental log2FC and used to train the other model. Parameters of imputer, scaler, selector and model are optimized using the software tool *auto-sklearn* with one iteration. The process should be iterated for a given number n of times, e.g. for 30 cycles to evaluate the convergence of the two models using autocorrelation (Spearman correlation between prediction of the current cycle and that of previous one cycle) and the model performance using Spearman correlation. The sum of predicted log2FC from two models are compared to the experimental log2FC error value as evaluation of the summed model.

### Model reduction

Given the top 30 features ranked by SHAP values, gRNA and PAM sequence single nucleotide features, 30nt extended sequence dinucleotide features, distance to start codon (percentage), four thermodynamic features, and off-target features can be selected for model reduction. Using single nucleotide features as a basic feature set, combinations of different numbers of other features can be added to the basic set to train models with a number n of e.g. 10 iterations while gene features remain unchanged. After selecting a reduced feature set, the iteration times with the best performance of the summed model among a number of e.g. 30 iterations can be selected as an appropriate number. The reduced model was adopted into a webpage tool using flask (version 1.1.2). Base preference was defined by the base frequency difference at each position between gRNAs with the lowest 20% log2FC and all gRNAs.

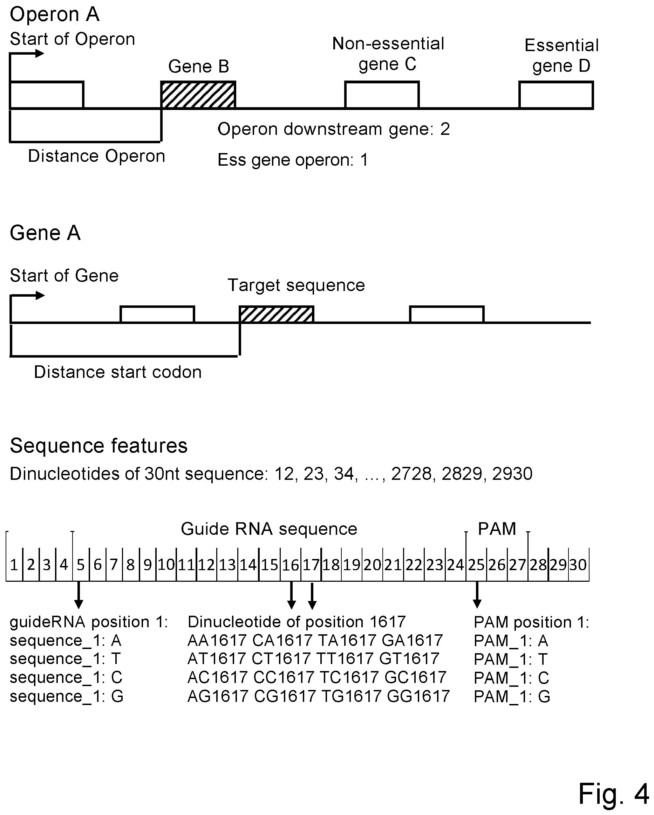

Figure 4 provides an explanation of the genomics and sequence features.

An exemplary operon A has, from the start of the operon a specific distance to a sequence of a related gene B, at least one non-essential gene C and at least one essential gene D.

An exemplary gene A comprises a target RNA sequence, which provides a genetic code, i.e. a codon. The starting point of the target sequence is located in a specific distance from the start of the RNA sequence of the related gene A.

A sequence comprises specific features, including a guide RNA (gRNA) sequence and a Protospacer Adjacent Motif (PAM). The PAM is a 2 to 6-base pair DNA sequence immediately following the DNA sequence targeted by the Cas9 nuclease in the CRISPR bacterial adaptive immune system.

The method for prediction of the targeting efficiency of guides targeting a gene of interest makes use of such features related in particular to a gene of interest and the target sequence. In the following, a set of gene intrinsic features, which are not modifiable and fixed by the characteristic of the gene of interest, and a set of guide features, which are modifiable in the design process, are described. Those features had been found having a significant effect on the location of a target and can be used as a guide in this regard.

**Examples of gene intrinsic features:**

| *Feature name* | *Explanation* | *Feature type* | *Number of features* |
|---|---|---|---|
| Gene id | Locus tag | Categorical | 1 |
| Gene length | Gene length (bp) | Numeric | 1 |
| Gene CG content | CG content of targeting gene (%) | Numeric | 1 |
| Distance operon | Distance of targeting gene to start of operon (bp) | Numeric | 1 |
| Distance operon perc | Distance of targeting gene to start of operon relative to operon (%) | Numeric | 1 |
| Operon downstream genes | Number of downstream genes in the same operon | Numeric | 1 |
| Ess gene operon | Number of downstream essential genes in the same operon | Numeric | 1 |
| Gene expression min | Maximal expression leven from OD0.1 to OD when the cells were collected | Numeric | 1 |
| Gene expression max | Maximal expression leven from OD0.1 to OD when the cells were collected | Numeric | 1 |
| Dataset | Origin of dataset | Categorical | 1 |

**Examples of Guide features:**

| Feature name | Explanation | Feature type | Number of features |
|---|---|---|---|
| Guide GC content | GC content of guide RNA (%) | Numeric | 1 |
| Distance start codon | Distance to start codon (bp) | Numeric | 1 |
| Distance start codon perc | Distance to start codon relative to gene length (%) | Numeric | 1 |
| Coding strand | Whether guide RNA targets coding strand | Categorical | 1 |
| Intergenic | Whether guide RNA targets intergenic regions | Categorical | 1 |
| Homopolymers | Length of longest consecutive nucleotides (nt) | Numeric | 1 |
| MFE hybrid full | Minimum free energy of hybridization of targeting DNA and guide RNA | Numeric | 1 |
| MFE hybrid seed | Minimum free energy of hybridization of 3' 8nt region of targeting DNA and guide RNA | Numeric | 1 |
| MFE homodimer guide | Minimum free energy of guide RNA homodimer | Numeric | 1 |
| MFE monomer guide | Minimum free energy of guide RNA monomer | Numeric | 1 |
| Off target | Number of potential off-targets with respective 90-100%, 80-90%, 70-80%, 60-70% similarities | Numeric | 4 |
| PAM sequence | One-hot-encoded 3nt PAM sequence | Categorical | 12 |
| gRNA sequence | One-hot-encoded 20 nt guide RNA sequence | Categorical | 80 |
| 30nt extended sequence dinucleotide | 16 possibilities for each dinucleotide (-4nt from 5' end to +6 nt of 3' end of guide RNA | Categorical | 464 |

The conventional features are extended in the above example to 580 sequence features, thermodynamic features, genomic features, and transcriptomic features. The sequence features comprises guide RNA and PAM single nucleotide sequences and 30nt extended sequence dinucleotide features. Thermodynamic features include minimum free energy of guide RNA monomer, guide RNA homodimer, and targeting DNA and guide RNA hybrid. The genomics features describe gene and operon organization, e.g. distance to start codon; and transcriptomic features describe the gene expression level. Including these features helps to assess the structural stability upon target recognition, the accessibility of guide RNA, the polar effect and other confounding effects.

When integrating data of the two different sets of CRISPRi screen data and the selected 580 features, a predictive random forest regression model can be optimized using automated machine learning (autoML). However, despite that ensemble method helps to avoid overfitting, increasing ensemble size led to no significant improvement.

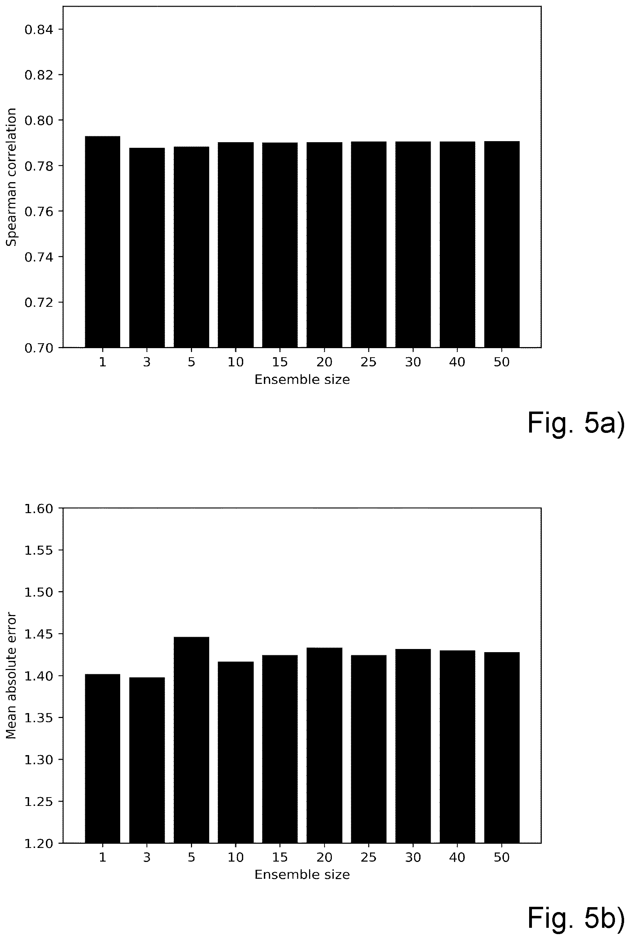

This can be seen in figure 5a) presenting a diagram showing the effect of the ensemble size on the Spearman correlation. The Spearman correlation achieved in the exemplary embodiment is 0.7873.

Figure 5b) is a diagram showing the effect of the ensemble size on the mean absolute error of prediction. It can be seen that the ensemble size has no significant effect on the prediction error.

Given two distinct peaks in the log2FC distribution and the need of weighing guide RNA usability, it is advisable to further partitioning the guide RNAs into strong and weak depletion classes using KMeans clustering for evaluation purposes.

Using the SHAP value various effects led to a stronger depletion, such as targeting coding strand, higher expression level, larger number of downstream essential genes in the same operon, and smaller distance to start codon. This indicates the ability of revealing meaningful design rules for CRISPRi. A number of top features with highest prediction attributions are gene-specific, underlining the confounding effects of genomic and transcriptomic features. These top features are for example coding strand, gene expression max, ess gene operon, dataset, gene expression min, gene length, distance start codon, distance operon, gene GC content and gene ID. Eight of these 10 top features are gene specific.

The attribution of gene-specific features confirms that the depletion-measured by log2FC is a combination of gene and guide effects. Therefore, segregating guide effects from log2FC suggests a more reliable guide RNA efficiency prediction.

When training a guide model and a gene model in an automated machine learning algorithm with e.g. the selected 570 guide and 10 gene features respectively through an iterative process and comparing the sum of predicted values from both models the experimental log2FC resulting in Spearman correlation 0.7962 and AUC value 0.91 were achieved.

To improve the interpretability of other guide features, guides targeting the 'coding_strand' were removed from the set of selected guides because the guide model heavily relied on it with its SHAP value 12-fold higher than the second feature. The retraining showed early convergence (6th cycle) and stable performance throughout the iterations with the expected weaker prediction (Spearman correlation 0.7547 and AUC value 0.88).

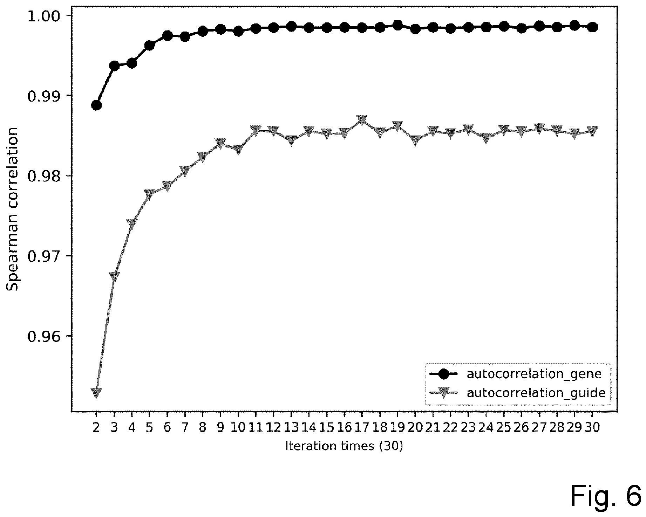

This can be seen in Figure 6 showing an exemplary diagram of the Spearman correlation of the autocorrelation of gene features and guide intrinsic features over the times of iteration.

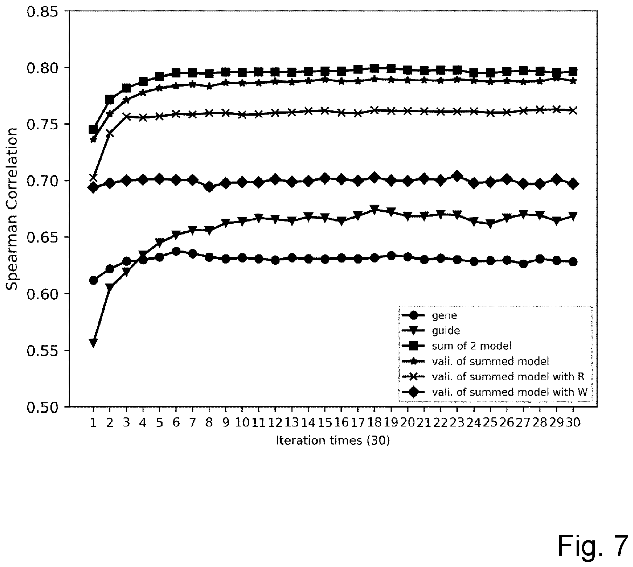

Accordingly, Figure 7 presents an exemplary diagram of the Spearman correlation of the autocorrelation of gene features and guide intrinsic features, the sum of the two models, the validation of the summed model and the validation of the summed model with the first set of CRISPRi screen and with the the second set of CRISPRi screen over the times of iteration.

Both Figures 6 and 7 presents that the autocorrelation showes early convergence of two models and Spearman correlation presented steady model performance during iterations.

Given the significant quantitative difference in feature contributions the number of guide features are reduced with a bottom-up approach. The model performed the best (Spearman correlation 0.7567, AUC value 0.87) with only sequence features, distance to start codon, GC content of gRNA, and minimum free energy of seed region DNA:RNA hybridization, gRNA homodimer and gRNA monomer.

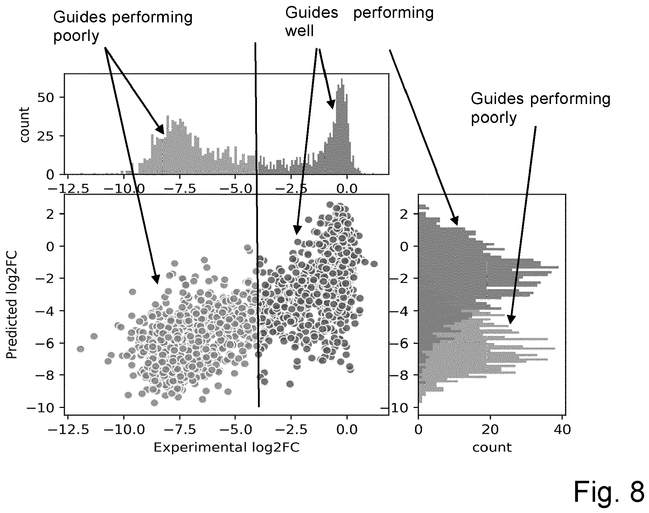

Figure 8 is a diagram of the experimental log2FC value compared to the known log2FC for guides performing well versus guides performing poorly based on clustering the logFCs. It is preferred to discriminate good guides from bad guides e.g. in the illustrated way with appropriate tools.

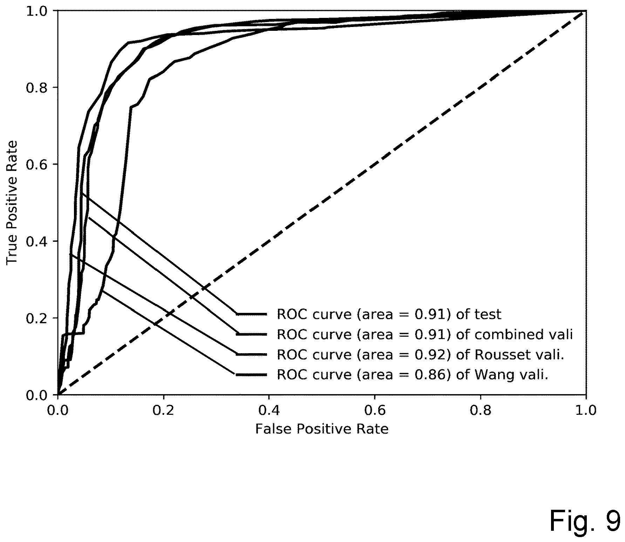

From Figure 9 showing a diagram of the true positive rate over the false positive rate using a first set of CRISPRi screen data (Wang), a second set of CRISPRi screen data (Rousset), a combined validation and a test using the above mentioned reduced set of selected features with both sets of CRISPRi screen data. It can be seeen that the reduced model with 561 features after 10 iterations demonstrated high prediction accuracy.

Interestingly, 6 of the top 10 features from the reduced guide model are sequence features near the PAM region corresponding to base preference and some effects differed from CRISPR/Cas9 system: guanine downstream of the PAM and adenine at the 3' end of guide RNA reduced guide RNA efficiency while cytosine at the N-position of PAM or downstream of the PAM, and two cytosines at the 3' end of guide RNA and the N-position of PAM increased guide RNA efficiency. This enables extracting distinct rules for CRISPRi guide RNA design.

The models can be validated by silencing GFP. Quantification of GFP signals avoids confounding gene-specific effects and reports the direct silencing efficiencies.

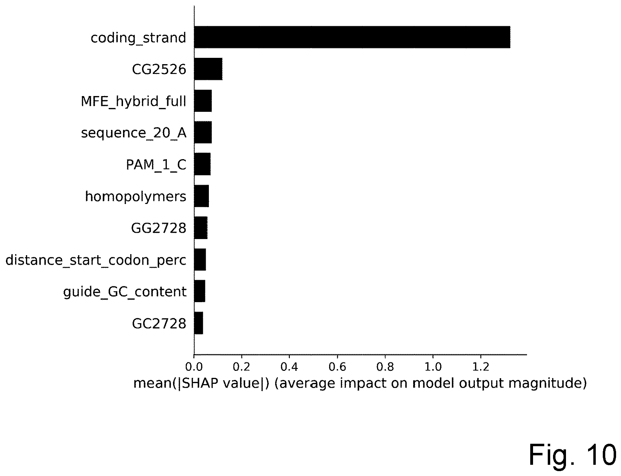

Figure 10 presents a table of the SHAP values of summed model with the feature "coding strand". It can be taken from this experiment, that the festure "coding strand" has a significant average impact on the magnitude of the model output compared to the other most relevant features like CG2526, MFE hybrid full, sequence_20_A, PAM_1_C, homopolymers, GC2728, Distance start codon perc, Guide GC content and GC2728.

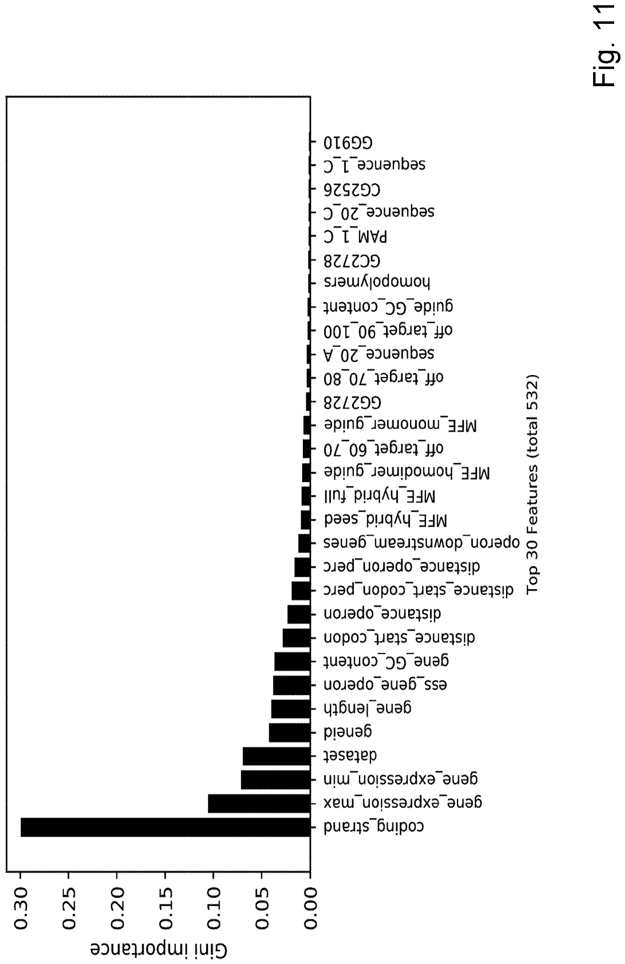

Figure 11 presents an exemplary table of the Gini importance of the 30 most relevant gene intrinsic features and guide features for E. coli related embodiment.

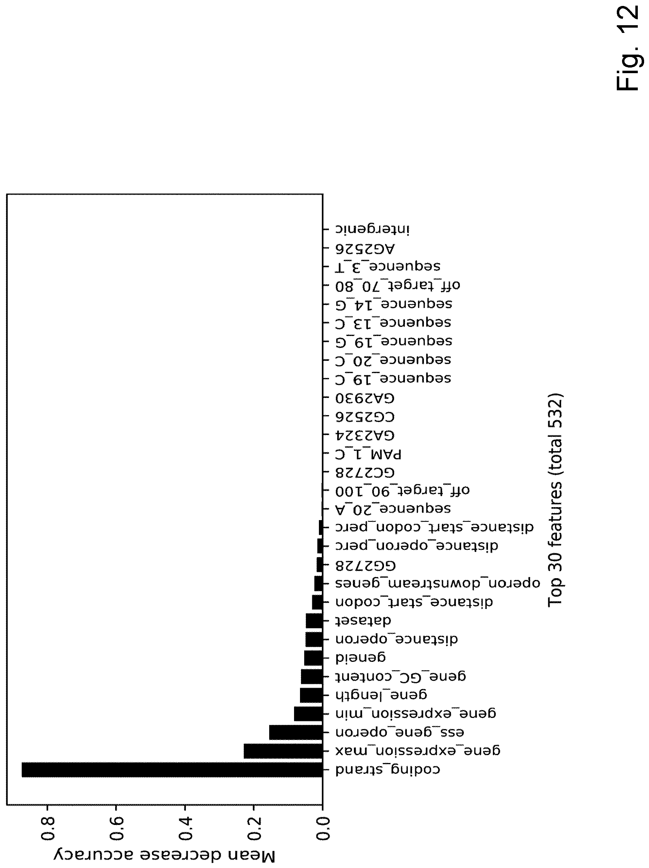

Figure 12 presents an exemplary table of the mean decrease accuracy of the 10 most relevant gene intrinsic features and guide features for E. coli related embodiment.

Due to the overwhelming effect of the feature "coding strand" compared to the other most relevant features, this feature is discarded in the following experiment.

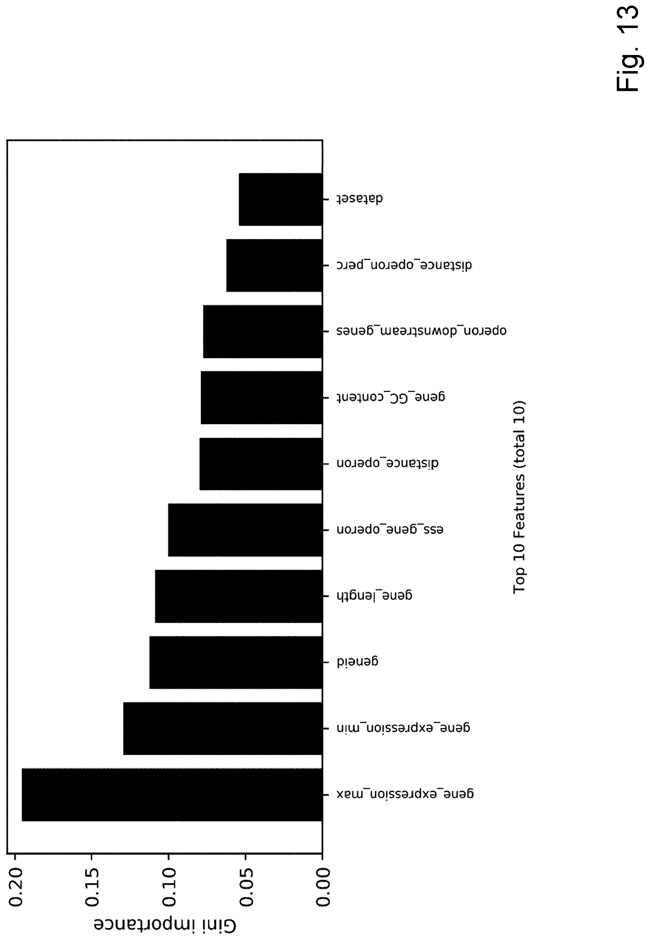

Figure 13 presents an exemplary table of the Gini importance of the 10 most relevant gene intrinsic features for E. coli related embodiment excluding the feature "coding strand" for 17 iterations.

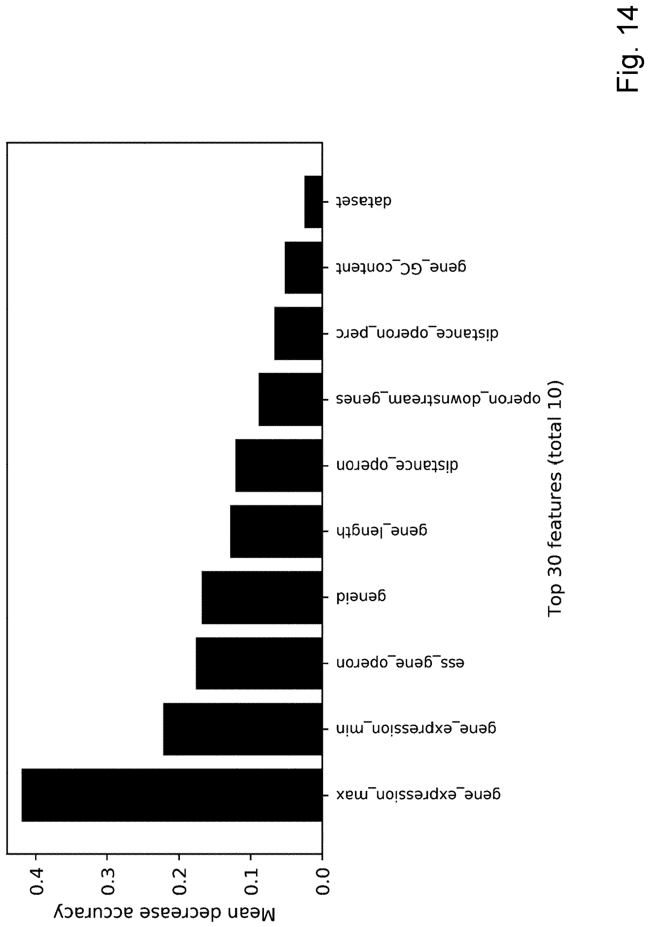

Figure 14 presents an exemplary table of the mean decrease accuracy of the 10 most relevant gene intrinsic features for E. coli related embodiment excluding the feature "coding strand" for 17 iterations.

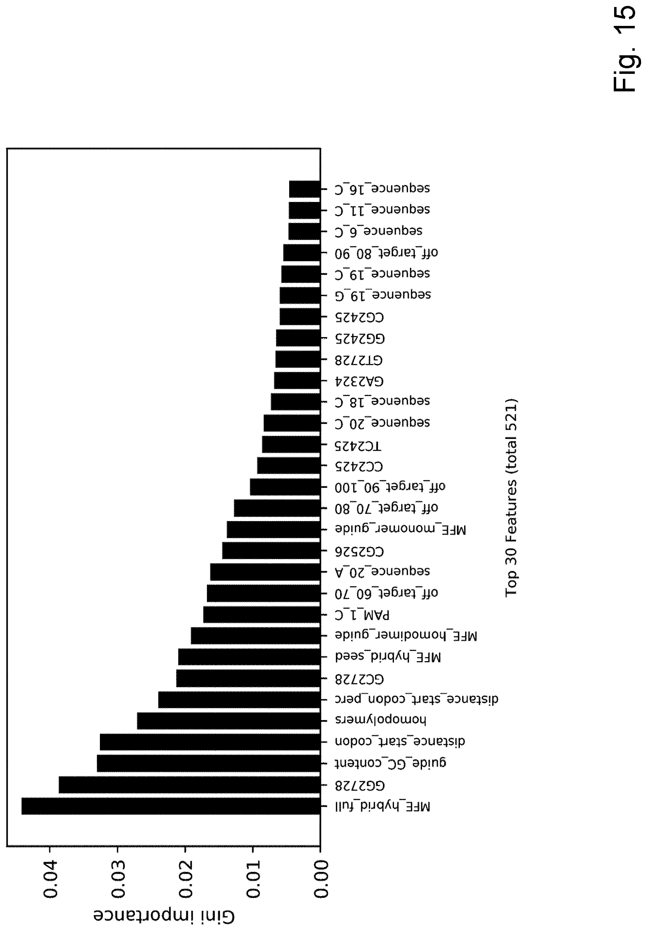

Figure 15 presents an exemplary table of the Gini importance of the 30 most relevant guide features for E. coli related embodiment excluding the feature "coding strand" for 17 iterations.

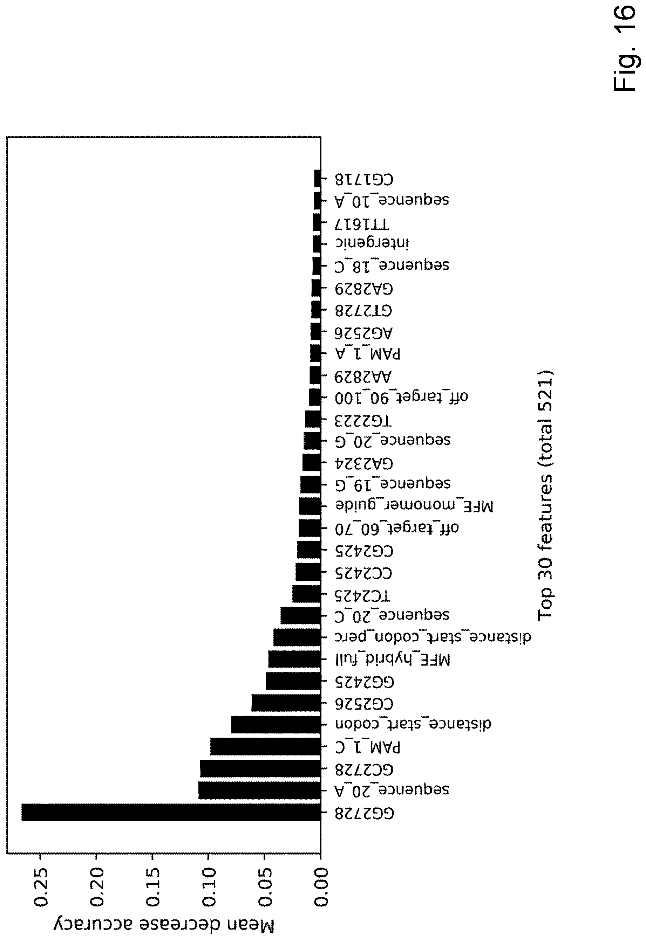

Figure 16 presents an exemplary table of the mean decrease accuracy of the 30 most relevant guide features for E. coli related embodiment excluding the feature "coding strand" for 17 iterations.

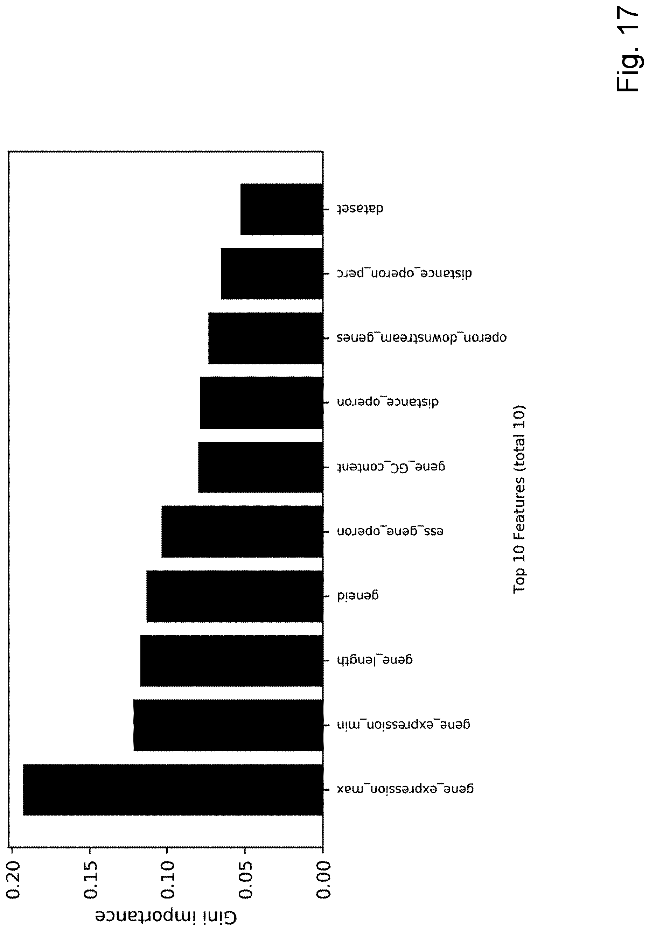

In comparison to the result for 17 iterations, the most relevant features for 10 iterations excluding the feature "coding strand" are shown in the following Figures 17 to 10.

Figure 17 presents an exemplary table of the Gini importance of the 10 most relevant gene intrinsic features for E. coli related embodiment excluding the feature "coding strand" for 17 iterations.

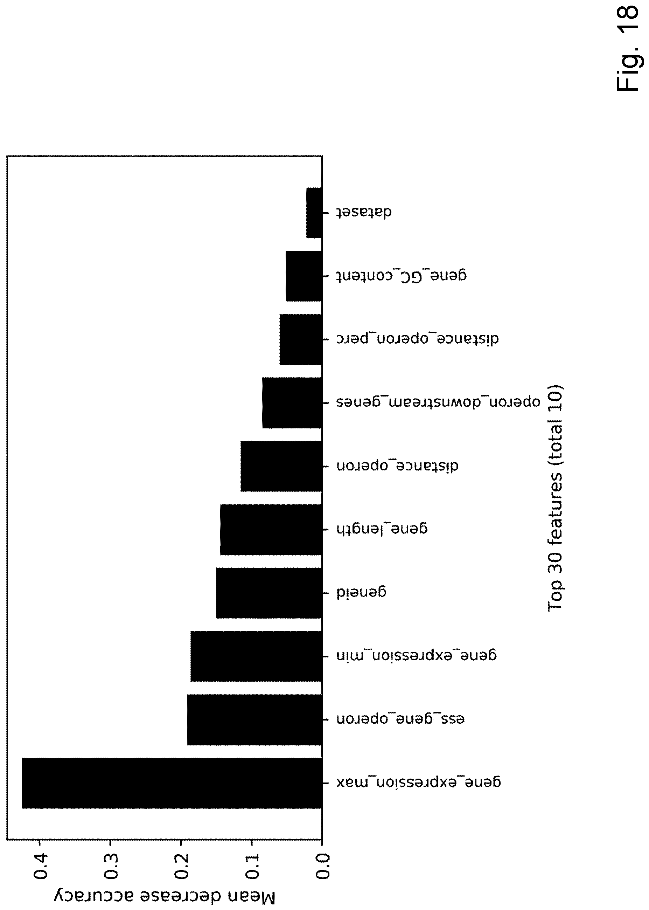

Figure 18 presents an exemplary table of the mean decrease accuracy of the 10 most relevant gene intrinsic features for E. coli related embodiment excluding the feature "coding strand" for 17 iterations.

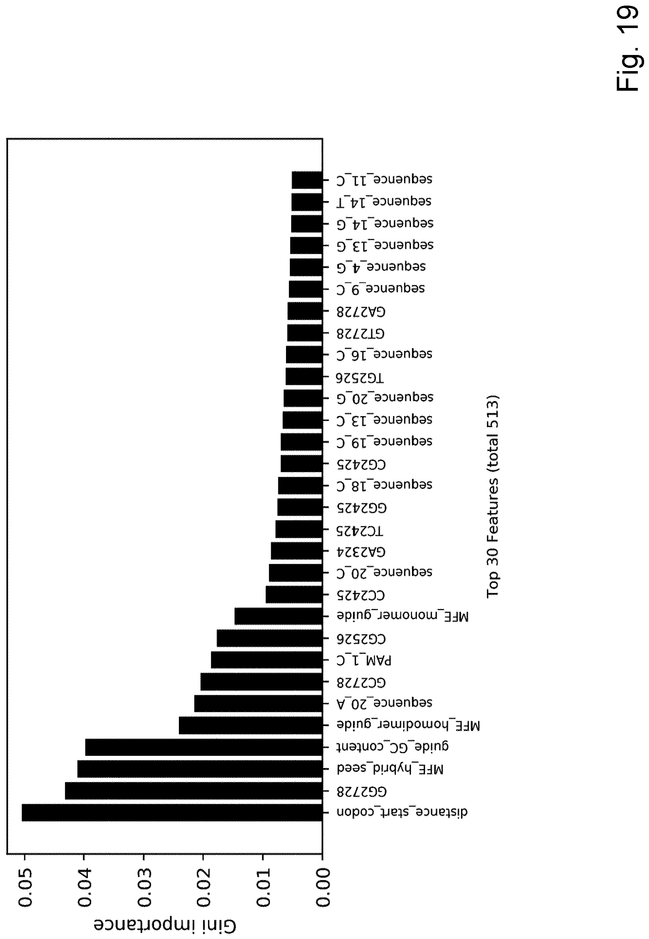

Figure 19 presents an exemplary table of the Gini importance of the 30 most relevant guide features for E. coli related embodiment excluding the feature "coding strand" for 17 iterations.

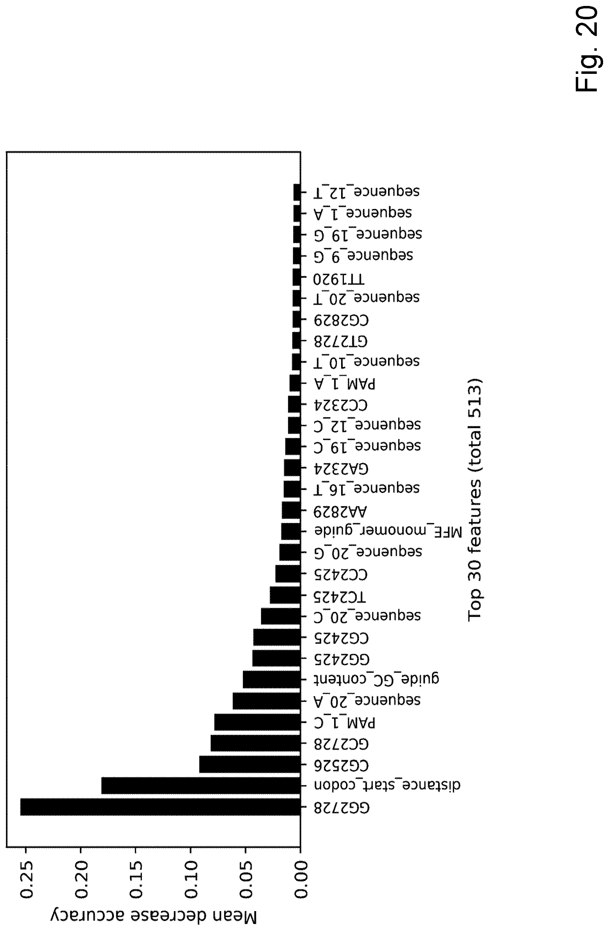

Figure 20 presents an exemplary table of the mean decrease accuracy of the 30 most relevant guide features for *E*. *coli* related embodiment excluding the feature "coding strand" for 17 iterations.

It can be seen that the combination of various feature types plays a role in training a predictive model. The expression level is of significance in log2FC prediction. The number of essential genes downstream of targeting genes in the same operon is more important than merely the number of downstream genes. The contribution of the dataset feature corresponds to the qualitative difference between two datasets used in the exemplary embodiment. The negative correlation between mRNA stability and length offers a potential explanation to that the bigger distance from targeting gene to start of the operon lowers log2FC.

The distance from targeting sequence to start codon and the feature of guanine at the position of +1 downstream of PAM ("GG2728") appears to be a relevant feature to be selected, while the result of feature "sequence_20_A" and "GG2425" have negative effects on guide efficiency. The feature "AT2425" is also relevant in the example.

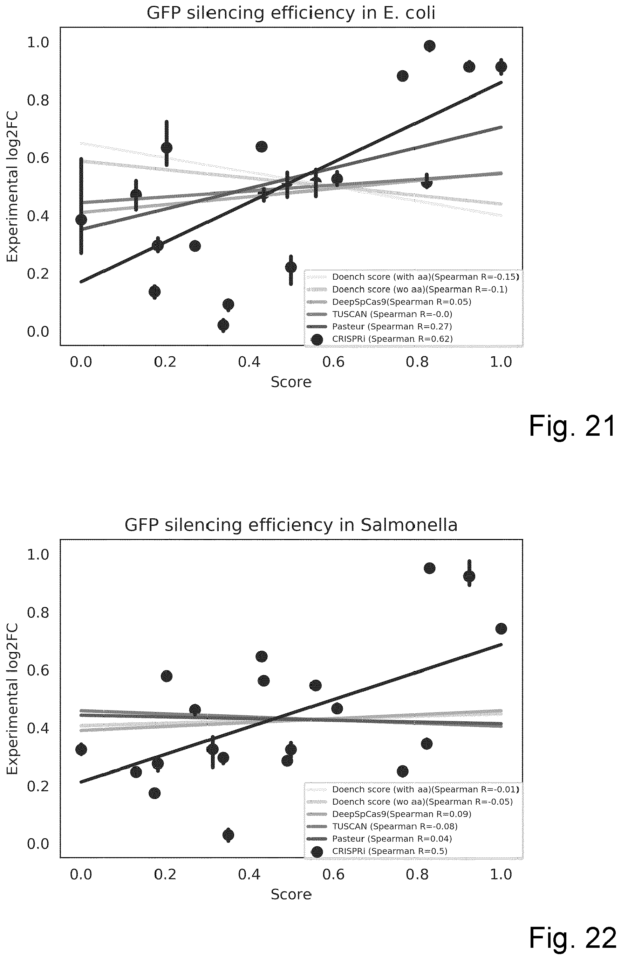

Figure 21 presents an exemplary plot the predicted activity versus the measured activity in *E*. *coli.* A set of sgRNAs were designed to target the GFP gene in different loccations. Each sgRNA was then tested by expressing each sgRNA along with dCas9 and GFP in E. coli and assessing fluorescence by flow cytometry analysis. Each component was expressed from a plasmid from constitutive promoters. The experimental log2FC was based on the extent of gene repression compared to a non-targeting control. Error bars represent the standard deviation from three independent experiments starting from separate colonies. Each algorithm was then used to predict the relative activity of each sgRNA. The scores from the herein-presented algorithm are plotted below. The line of best fit for this algorithm and other available algorithms are also plotted. The presented algorithm yielded the highest predictive ability based on the Spearmann coefficient.

Figure 22 presents an exemplary plot comparing the predicted activity to measured activity in *S*. *enterica.* The predicted activity was from the herein-presented algorithm using the screening data from *E*. *coli.* The measured activity came from using the same sgRNAs and constructs from Figure 21 in *S*. *enterica.* Error bars represent the standard deviation from three independent experiments starting from separate colonies. Each algorithm was then used to predict the relative activity of each sgRNA. The presented algorithm again could predict measured activity, albeit with a lower Spearmann coefficient than that from the measuremens in *E*. *coli.*

## Claims

1. Method for prediction of the targeting efficiency of guides targeting a gene of interest, said guides locating a target in a gene sequence, by evaluating of data provided by screens providing values of targeting characteristics of guides, **characterized by** the steps of
a) Selecting a set of guides along with gene intrinsic features related to a gene of interest and guide features;
b) Inputting the selected features into an automated machine learning algorithm and
c) Calculating an estimate of targeting efficiency of said selected guides by use of the automated machine learning algorithm.

2. Method according to claim 1, **characterized by**
- Training of parameters of the automated machine learning algorithm by calculating at least one error value by comparing the calculated estimate of targeting efficiency of a set of selected guides with a known level of targeting efficiency of said selected guides,
- Adapting the parameters as a function of the calculated error value and
- Iteratively repeating the step of calculating an estimate of targeting efficiency by use of the automated machine learning algorithm in order to adapt the parameters for reducing the at least one error value.

3. Method according to claim 2, **characterized in** adapting the parameters by Random Forest Regression.

4. Method according to one of claims 2 or 3, **characterized by** performing the step of training of parameters of the automated machine learning algorithm by use of at least two sets of screening data which are obtained by different methods.

5. Method according to claim 4, **characterized by** using a first set of screening data which first set is obtained by only including guide RNAs for targeting the coding strand of the gene of interest and collected in only one specific growth phase and using a second set of screening data which second set is obtained by including guide RNAs for targeting the coding strand and the template strand.

6. Method according to one of claims 2 to 5, **characterized by** classifying the guide RNA provided in at least one set of screening data, which is used for evaluating the parameters of the automated machine learning algorithm, into a group of guide RNAs providing strong depletion and a group of guide RNA providing weak depletion.

7. Method according to one of the preceding claims, **characterized in that** the set of features selected in step a) comprising the feature of an RNA expression level.

8. Method according to one of the preceding claims, **characterized in that** the gene intrinsic features selected in step a) for including in the set of features comprising at least one of the locus tag, the length of the targeting gene of interest, the guanine-cytosine (GC) content of the targeting gene of interest, the distance of the targeting gene of interest to start of an operon, the distance of the targeting gene of interest to start of the operon relative to the operon length, the number of downstream genes in the same operon, the number of downstream essential genes in the same operon, the minimal expression level from a first concentration to a second concentration of cells when the cells were collected, and the maximal expression level from a first concentration to a second concentration of cells when the cells were collected.

9. Method according to one of the preceding claims, **characterized in that** the guide features selected in step a) for including in the set of features comprising at least one of the guanine-cytosine (GC) content of the guide RNA, the distance to start a codon, the distance to start the codon relative to the length of the gene of interest, the function of a guide RNA of being able to target a coding strand, the function of a guide RNA being able to target intergenic regions, the length of the longest consecutive nucleotides, the minimum free energy of hybridization of targeting DNA and guide RNA, the minimum free energy of hybridization of a specific region of targeting DNA and guide RNA, the minimum free energy of guide RNA homodimer, the minimum free energy of guide RNA monomer, the number of potential of-targets with specific similarities, at least one PAM sequence, at least one guide RNA sequence, and a plurality of possibilities for extended sequence dinucleotide.

10. Method according to one of the preceding claims, **characterized in that** the automated machine learning algorithm comprising two models separately trained from each other, wherein the first of the two models using the selected gene intrinsic features and the second of the two models using the selected guide features.

11. Method according to one of the preceding claims, **characterized by** evaluating of data provided by Clustered Regularly Interspaced Short Palindromic Repeat (CRISPR) screens, in particular CRISPR interference (CRISPRi) screens or CRISPR activation (CRISPRa) screens.

12. Computer program comprising instructions which, when the program is executed by a computer, cause the computer to carry out the steps of the method of one of claims 1 to 11.

13. Data processing apparatus comprising means for carrying out the steps of the method according to one of claims 1 to 11.
